# EUROPEAN PATENT SPECIFICATION

(11) **EP 2 477 545 B1**
(45) Date of publication and mention of the grant of the patent: **18.09.2013**
(21) Application number: 10785391.3
(22) Date of filing: 22.11.2010
(51) Int. Cl.: A61B 5/107

(54) **DEVICE**
Vorrichtung
Dispositif

(30) Priority: 24.11.2009 EP 09176899
(43) Date of publication of application: 25.07.2012
(73) Proprietor: Unilever PLC, A Company Registered in England and Wales under Company no. 41424, London, Greater London EC4Y 0DY (GB); Unilever N.V., 3013 AL Rotterdam (NL)
(72) Inventor: BAILEY, Peter, Lawrence, Wirral Merseyside CH63 3JW (GB); BRADY, Daniel, Old Windsor Berkshire SL4 2JW (GB); CHANDLER, Matthew, Daniel, Cambridge Cambridgeshire CB1 3RD (GB); COLLIER, Nicholas, John, Burwell Cambridgeshire CB25 0BB (GB); COPELAND, Thomas, John, Hector, Cambridge Cambridgeshire CB4 2PA (GB); SAYERS, Stephen, Anthony, Aston Clinton Buckinghamshire HP22 5EG (GB)
(74) Representative: Newbould, Frazer Anthony
(86) International application number: PCT/EP2010/067906
(87) International publication number: WO 2011/064166

(56) References cited:
- EP-A1- 1 691 195
- WO-A1-2005/106465
- WO-A1-2007/108318
- DE-A1- 19 924 741
- JP-A- 63 163 143
- US-A- 4 061 022
- US-A- 4 628 742

## Description

The present invention relates to a device for measuring hair damage.

JP 63 163 143 A discloses a device for measuring the degree of hair damage. WO 2007/108318 A1 discloses a device for measuring hair characteristics.

Despite the prior art there remains the need for devices for measuring hair damage.

Accordingly, and in a first aspect, the present invention provides a device according to claim 1.

Preferably, the clip comprises a pair of opposing tongues pivotally attached to each other.

Preferably, the tongues are maintained in a closed position by a spring.

Preferably, the tongues comprise an overhang which extends beyond the pivot and so provides a means for opening the clip against the closing pressure of the spring.

Preferably, the mandrel and the clip are attached to one another such that a subject's hair may be wound around the mandrel and maintained in place by the clip. Preferably, the mandrel is attached to the clip at one end only. The mandrel and clip combination thus provide a gap between mandrel and tongues to allow the hair to be wrapped around the mandrel when the clip is opened.

Preferably, the mandrel comprises a groove. Such a groove assists to provide an aligned portion of hair thus providing more accurate friction measurement. Preferably, the mandrel comprises a series of grooves for maintaining hair aligned and on the mandrel during use.

Preferably, the device comprises means for measuring friction between the superimposed hair fibres. An example of such a means is a load cell.

Preferably, the device comprises a handle and a shaft, the shaft having a handle end and a head end, the head end being placed against the head during use to maintain the relative position of the device during use.

Preferably, the mandrel is moved along the shaft when measuring friction between the hair fibres as they are passed over each other. Preferably, the device comprises a motor for automatically moving the mandrel clip along the shaft at a stead rate. This provides a calibrated measuring capability.

Preferably, the mandrel and clip is attached to the device by a magnet. Preferably, the mandrel is attached directly to a trolley on the device.

Preferably, the trolley is drivable along the shaft on the device. During use, the trolley is driven from a head end of the shaft to a handle end of the shaft by a motor. Preferably, the means for measuring friction measures the force required to drive the trolley from the head end of the shaft to the handle end of the shaft. This force is then indicated to the user. During use, the subject's hair is wound around the mandrel and clipped into place by the clip such that hair passes through the clip, around the mandrel and then back through the clip again. The mandrel and clip are then attached to the trolley ready for use.

The device according to the invention is thus able to provide a measure of the condition of the hair fibres by rubbing them over each other and measuring the force required to do this over a set distance. When the hair fibres are in good condition they are smoother and rub over each other more easily thus requiring less force to pass over one another. When the hair fibres are in a poor condition they are less easily rubbed over one another and so require more force. Accordingly, the device is able to inform the user of the condition of their hair, particularly with regard to fibre damage.

Preferably, the device comprises a power supply. Preferably, such a power supply is rechargeable.

In use the mandrel and clip are attached to one another and are preferably detached from the remainder of the device. The clip is opened and the subject's hair is placed between the mandrel and the clip and then wound around the mandrel such that the hair is passed back towards the hair root, and head. The clip is then closed. The mandrel and clip are then attached to a trolley on the device. The trolley is attached to a drive shaft and, when activated, is driven down the shaft away from the subject's head. Through this process the user's hair is passed through the closed clip and around the mandrel and back through the clip. Accordingly, the hair is passed over itself and so hair travelling in one direction is pressed against hair moving in the opposite direction by the closed clip. Once the trolley reaches the handle end of the shaft a reading is provided on the feedback indicator.

In a second aspect there is provided a method for determining the state of a user's hair by drawing the hair fibres against one another and along the general length of the hair and measuring the friction. Preferably, a lock of hair is folded over itself and drawn along its own length such that the hair towards the tip is drawn backwards over the hair towards the scalp.

An embodiment of the device is further described with reference to the following non-limiting figures in which:

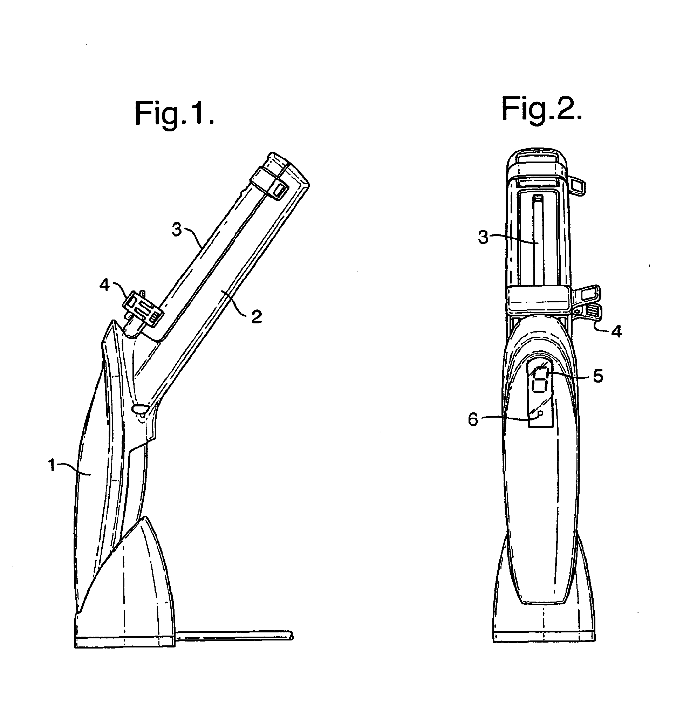

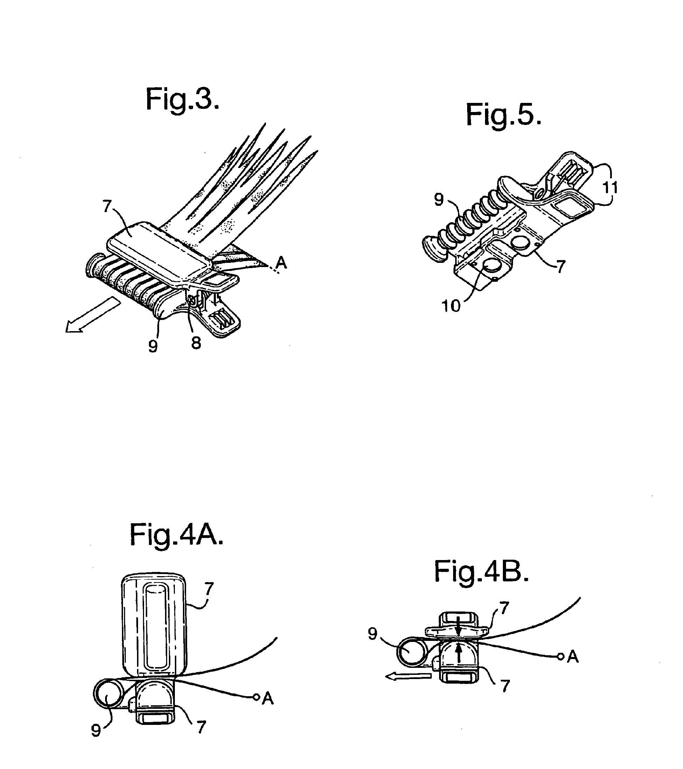

Figure 1 is a side elevation of a device; Figure 2 is a front elevation of the same device; Figure 3 is an isometric view of the mandrel; Figures 4a and 4b are the same view of the clip; and Figure 5 is a view of the underside of the mandrel clip.
   In Figure 1 is a side view of a device with a battery housing 1 and a handle 2. The handle has a causeway 3 along which travels a motorized mandrel clip 4. In use, the subject's hair is passed under the clip 4 and around the mandrel so that the hair tips are folded back towards the hair root. The device also comprises a load cell (not shown) to measure the force required to pull the mandrel clip 4 along the causeway.
   The force measured is indicated on the LCD display 5 on the housing. Display 6 indicates that the device is turned on.
Figure 2 is a front elevation of the device illustrated in Figure 1.
Figure 3 is a close up view of the mandrel clip seen in Figures 1 and 2. The mandrel clip comprises a clip which is in the form of a pair of opposing tongues 7 (only one shown) which are pressed together and maintained together by a spring (not shown) at their pivot 8. The mandrel clip also comprises a mandrel 9 around which hair is wrapped back on itself.
Figure 4a shows the clip in an open configuration and Figure 4b shows the same clip in a closed configuration. In each figure can be seen the mandrel 9 around which hair is wound such that it passes back onto itself towards the root A. The clip is closed and the mandrel is passed along the hair fibres away from the root so that the hair from the tip is passed back over the hair from the root.
Figure 5 shows the mandrel 9 and the bottom of one of the tongues 7. The clip is openable by squeezing the flanges 11 towards one another. The mandrel has a pair of magnets 10 by which the mandrel is attachable to the remainder of the device.

## Claims

1. Device for measuring hair damage comprising a handle (2) and a shaft (3) and a mandrel (9) a clip (4) and a feedback indicator, wherein the mandrel and clip are attachable to the shaft (3) and the mandrel is drivable along the length of the shaft to measure the friction of a lock of hair which is wound around the mandrel and back onto itself and held in place by the clip such that fibres in the lock of hair are rubbed against themselves along the general direction of the hair fibres.

2. Device according to claim 1 wherein the clip (4) comprises a pair of opposing tongues (7) pivotally attached to each other.

3. Device according to claim 2 wherein the tongues (7) are maintained in a closed position by a deformable spring.

4. Device according to any preceding claim wherein the mandrel (9) and the clip (4) are attached to one another such that a subject's hair may be wound around the mandrel and maintained in place by the clip.

5. Device according to any preceding claim wherein the mandrel (9) comprises a groove.

6. Device according to any preceding claim comprising a load cell for measuring the force required to pass the hair fibres along one another.

7. Device according to any preceding claim wherein the mandrel (9) is attached at one end to the clip (4).

8. Device according to any preceding claim where the mandrel (9) and clip (4) are attached to a trolley on the device.

9. Device according to claim 8 wherein the trolley is located on a drive shaft.

10. Device according to claim 8 or 9 wherein the mandrel and clip is attachable to the trolley by magnetic means (10).

11. Use of a device according to any preceding claim for measuring the state of health of a user's hair.

## Patentansprüche

1. Vorrichtung zum Messen von Haarschaden, die einen Griff (2), einen Schaft (3), einen Drehstift (9), eine Klammer (4) und eine Rückmeldungsanzeigeeinrichtung umfasst, wobei der Drehstift und die Klammer an dem Schaft (3) befestigbar sind und der Drehstift entlang der Länge des Schafts antreibbar ist, um die Reibung einer Haarlocke zu messen, die um den Drehstift und auf sich selbst zurück gewickelt ist und durch die Klammer festgehalten wird, so dass Fasern in der Haarlocke entlang der allgemeinen Richtung der Haarfasern gegeneinander gerieben werden.

2. Vorrichtung nach Anspruch 1, wobei die Klammer (4) ein Paar gegenüberliegender Zungen (7), die kippbar aneinander befestigt sind, umfasst.

3. Vorrichtung nach Anspruch 2, wobei die Zungen (7) durch eine verformbare Feder in einer geschlossenen Position gehalten werden.

4. Vorrichtung nach einem der vorhergehenden Ansprüche, wobei der Drehstift (9) und die Klammer (4) aneinander befestigt sind, so dass das Haar einer Testperson um den Drehstift gewickelt und durch die Klammer festgehalten werden kann.

5. Vorrichtung nach einem der vorhergehenden Ansprüche, wobei der Drehstift (9) eine Kerbe umfasst.

6. Vorrichtung nach einem der vorhergehenden Ansprüche, die eine Wägezelle zum Messen der Kraft, die benötigt wird, um die Haarfasern aneinander vorbeizuführen, umfasst.

7. Vorrichtung nach einem der vorhergehenden Ansprüche, wobei der Drehstift (9) an einem Ende der Klammer (4) angebracht ist.

8. Vorrichtung nach einem der vorhergehenden Ansprüche, wobei der Drehstift (9) und die Klammer (4) an einem Wagen auf der Vorrichtung befestigt sind.

9. Vorrichtung nach Anspruch 8, wobei sich der Wagen auf einer Antriebswelle befindet.

10. Vorrichtung nach Anspruch 8 oder 9, wobei der Drehstift und die Klammer durch magnetische Mittel (10) an dem Wagen befestigbar sind.

11. Verwendung einer Vorrichtung nach einem der vorhergehenden Ansprüche zum Bestimmen des Gesundheitszustands des Haars eines Nutzers.

## Revendications

1. Dispositif de mesure de la dégradation des cheveux comprenant un manche (2) et une tige (3) ainsi qu'un mandrin (9), une pince (4) et un indicateur de réaction, dans lequel le mandrin et la pince peuvent être fixés à la tige (3) et le mandrin peut être entraîné le long de la tige afin de mesurer le frottement d'une mèche de cheveux qui est enroulée autour du mandrin puis sur elle-même et maintenue en place par la pince de sorte que les fibres de la mèche de cheveux se frottent les unes contre les autres le long de la direction générale des fibres capillaires.

2. Dispositif selon la revendication 1, dans lequel la pince (4) comprend deux languettes (7) fixées pivotantes l'une à l'autre.

3. Dispositif selon la revendication 2, dans lequel les languettes (7) sont maintenues dans une position fermée par un ressort déformable.

4. Dispositif selon l'une quelconque des revendications précédentes, dans lequel le mandrin (9) et la pince (4) sont fixés l'un à l'autre de sorte que les cheveux d'une personne puissent s'enrouler autour du mandrin et être maintenus en place par la pince.

5. Dispositif selon l'une quelconque des revendications précédentes, dans lequel le mandrin (9) comprend une rainure.

6. Dispositif selon l'une quelconque des revendications précédentes comprenant une cellule de charge destinée à mesurer la force nécessaire pour faire passer les fibres capillaires le long les unes des autres.

7. Dispositif selon l'une, quelconque des revendications précédentes, dans lequel le mandrin (9) est fixé par une première extrémité à la pince (4).

8. Dispositif selon l'une quelconque des revendications précédentes, dans lequel le mandrin (9) et la pince (4) sont fixés à un curseur situé sur le dispositif.

9. Dispositif selon la revendication 8, dans lequel le curseur se situe sur une tige d'entraînement.

10. Dispositif selon la revendication 8 ou 9, dans lequel le mandrin et la pince peuvent être fixés au curseur par un moyen magnétique (10).

11. Utilisation d'un dispositif selon l'une quelconque des revendications précédentes pour mesurer l'état de santé des cheveux d'un utilisateur.
